# EUROPEAN PATENT SPECIFICATION

(11) **EP 2 123 639 B1**
(45) Date of publication and mention of the grant of the patent: **26.03.2014**
(21) Application number: 07707645.3
(22) Date of filing: 29.01.2007
(51) Int. Cl.: C07D 213/64

(54) **PROCESS FOR PRODUCTION OF 2,3'-BIPYRIDYL-6'-ONE**
VERFAHREN ZUR HERSTELLUNG VON 2,3ý-BIPYRIDYL-6ý-ON
PROCÉDÉ DE FABRICATION DE LA 2,3'-BIPYRIDYL-6'-ONE

(43) Date of publication of application: 25.11.2009
(73) Proprietor: Fujifilm Finechemicals Co., Ltd., Hiratsuka-shi Kanagawa 254-0016 (JP); Eisai R&D Management Co., Ltd., Tokyo 112-8088 (JP)
(72) Inventor: Takayuki SONODA, Hiratsuka-shi, Kanagawa 254-0016 (JP); Taichi SHINTOU, Saitama 330-0854 (JP); Kenichi ONOUE, Hiratsuka-shi, Kanagawa 254-0016 (JP); Takashi NAGAYAMA, Hiratsuka-shi, Kanagawa 254-0016 (JP)
(74) Representative: HOFFMANN EITLE
(86) International application number: PCT/JP2007/051414
(87) International publication number: WO 2008/093392

(56) References cited:
- WO-A1-2004/009553
- JP-A- 01 031 768
- JP-A- 01 319 461
- JP-A- 2001 261 647
- JP-A- 2001 261 653
- JP-A- 2005 213 239
- JP-A- 2005 239 672
- PARRY, PAUL R.; WANG, CHANGSHENG; BATSANOV, ANDREI S.; BRYCE, MARTIN R.; TARBIT, BRIAN: "Functionalized Pyridylboronic Acids and Their Suzuki Cross-Coupling Reactions To Yield Novel Heteroarylpyridines", J. ORG. CHEM., vol. 67, 21 September 2002 (2002-09-21), pages 7541-7543, XP002632848, DOI: 10.1021/jo020388b

## Description

### TECHNICAL FIELD

The present invention relates to a production process of 2,3'-bipyridyl-6'-one that is a useful intermediate in the fields of pharmaceutical agents, agricultural chemicals, catalyst ligands, organic electroluminescent devices, electric mobiles, electronic photoreceptors, dyes, liquid crystals, solar cells and the like.

### BACKGROUND ART

2,3'-Bipyridyl-6'-one is a useful intermediate in a wide range of fields such as pharmaceutical agent, catalyst ligand, organic electroluminescent device and liquid crystal, and particularly in the field of pharmaceutical agents, is very useful as an intermediate of pharmaceutical agents for Parkinson's disease, migraine, epilepsy and neurogenic diseases such as multiple sclerosis (see, Patent Documents 1, 2, 7-10).

As for the production process of 2,3'-bipyridyl-6'-one, a process of coupling a 5-bromopyridine derivative with a 2-sulfonylpyridine derivative in the presence of an alkyl lithium such as butyl lithium or a Grignard reagent such as ethyl magnesium bromide is known (Patent Document 3). However, in this case, an expensive metal reagent is used or special equipment such as low-temperature reactor is required and because of these and other cost problems, the process is improper as an industrial production process.

On the other hand, as a production process of a 2,3'-bipyridyl-6'-ol derivative that is a tautomer of 2,3'-bipyridyl-6'-one (see, Non-Patent Document 1), there are known a process of coupling a 2-alkoxypyridine having a boron or tin atom at the 6-position with a 2-halogenated pyridine in the presence of a palladium catalyst (Patent Documents 4 and 5) and a process of coupling a pyridine derivative having a boron or tin atom at the 2-position with a 5-halogenated-2-alkoxypyridine (Patent Documents 2 and 6). However, in these processes, an expensive metal reagent such as palladium is used and there is also a problem of waste solution. Patent Documents 7-10 equally disclose different processes for the preparation of bipyridylderivatives by reaction with acetylpyridin precursors.
Patent Document 1: WO 03/047577
Patent Document 2: WO 01/96308
Patent Document 3: WO 04/009553
Patent Document 4: WO 01/81310
Patent Document 5: U.S. Patent 5,693,611
Patent Document 6: WO 01/27112
Patent Document 7: JP 2005/213 239
Patent Document 8: JP2001/261 653
Patent Document 9: JP 2005/239 672
Patent Document 10: 2001/261 647

Non-Patent Document 1: March's Advanced Organic Chemistry, 5th Ed., pp. 73-77, WILEY-INTERSCIENCE (2001) Also P. Parry et al., J. Org. Chem 2002, 67, 7541-7543 describes the synthesis of certain pyridylboranic acid derivatives and their reactions using borone derivatives.

### DISCLOSURE OF THE INVENTION

### PROBLEMS THAT THE INVENTION IS TO SOLVE

An object of the present invention is to provide a production process where 2,3'-bipyridyl-6'-one useful as an intermediate in the fields of pharmaceutical agents, agricultural chemicals, catalyst ligands, organic electroluminescent devices, electric mobiles, electronic photoreceptors, dyes, liquid crystals, solar cells and the like, particularly as an intermediate of pharmaceutical agents for Parkinson's disease, migraine, epilepsy and neurogenic diseases such as multiple sclerosis can be produced in high purity at low cost on an industrial scale without using an expensive catalyst or special equipment.

### MEANS FOR SOLVING THE PROBLEMS

As a result of intensive studies to attain the above-described object, the present inventors have found a synthesis method of 2,3'-bipyridyl-6'-one, which can solve those problems, and have accomplished the present invention. That is, the object of the present invention is attained by the following process.
<1> A process for producing 2,3'-bipyridyl-6'-one, comprising:
   reacting an acetylpyridine derivative represented by formula (I) with at least one of compounds represented by formulae (II) to (V) to synthesize a bipyridine derivative represented by the following formula (VI); and
   hydrolyzing the bipyridine derivative by one-pot preparation: 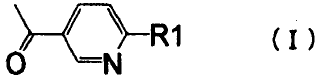 wherein R1 represents a hydroxyl group, an alkoxy group or a halogen atom; 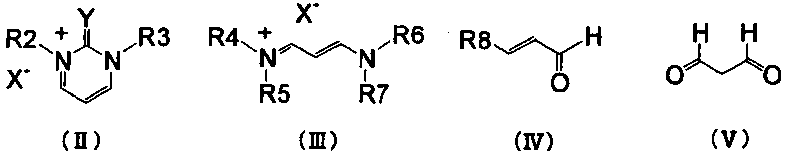 wherein each of R2 and R4 independently represents an alkyl group, an alkenyl group, an alkynyl group, an aryl group, a carbonyl group, a sulfonyl group, an amino group, a ureido group, a carbonylamino group, a sulfonylamino group, a cyano group or a heterocyclic residue;
   each of R3 and R5 to R7 independently represents an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a nitro group, a cyano group, a heterocyclic residue or a halogen atom;
   each of the pair of R4 and R5 and the pair of R6 and R7 may combine together to form a ring;
   R8 represents a hydroxyl group, an alkoxy group, an aryloxy group, a carbonyloxy group, a carbonyl group, a sulfonyl group, an amino group, a carbonylamino group or a sulfo group;
   X⁻ represents an arbitrary anion; and
   Y represents an oxygen atom, a sulfur atom, a selenium atom or -N(R9), wherein R9 represents a hydrogen atom, an alkyl group, an alkenyl group, an alkynyl group, an aryl group, a hydroxyl group, a carbonyl group, a sulfonyl group, an amino group or a heterocyclic residue; 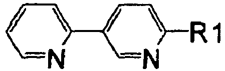 wherein R1 has the same meaning as above, and wherein a compound represented by formulae (II) to (V) is added in portions in the step of obtaining a bipyridine derivative from an acetylpyridine derivative, and wherein the number of additions of at least one of compounds represented by formulae (II) to (V) is 2 to 10, and second and subsequent additions of at least one of compounds represented by formulae (II) to (V) are conducted when the reaction ratio exceeds 45%.
<2> A process for producing 2,3'-bipyridyl-6'-on.e, comprising:
   a step of converting a nicotinic acid derivative represented by formula (VII) into nicotinoyl chloride;
   a step of reacting said nicotinoyl chloride with a malonic acid derivative represented by formula (VIII) to obtain a ketoester derivative represented by formula (IX) or (X);
   a step of hydrolyzing said ketoester derivative to obtain an acetylpyridine derivative represented by formula (I) ;
   a step of reacting said acetylpyridine derivative with at least one of compounds represented by formulae (II) to (V) to obtain a bipyridine derivative represented by formula (VI); and
   a step of hydrolyzing said bipyridine derivative,
   wherein each of the series of the steps from said nicotinic acid derivative to said acetylpyridine derivative and the series of the steps from said acetylpyridine derivative to 2,3'-bipyridyl-6'-one is performed by one-pot preparation: 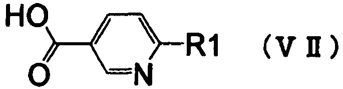 wherein R1 represents a hydroxyl group, an alkoxy group or a halogen atom; 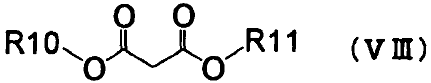
   wherein each of R10 and R11 independently represents a hydrogen atom, an alkyl group, an alkenyl group, an alkynyl group, an aryl group, a carbonyl group, a heterocyclic residue, an alkali metal atom, an alkaline earth metal atom, a typical metal atom, a transition metal atom or a nonmetallic atom, and R10 and R11 may combine to form a ring; 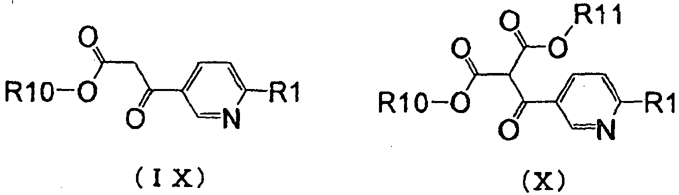
   wherein R1, R10 and R11 have the same meanings as above, and wherein a compound represented by formulae (II) to (V) is added in portions in the step of obtaining a bipyridine derivative from an acetylpyridine derivative, and wherein the number of additions of at least one of compounds represented by formulae (II) to (V) is 2 to 10, and second and subsequent additions of at least one of compounds represented by formulae (II) to (V) are conducted when the reaction ratio exceeds 45%.
<3> The process for producing 2,3'-bipyridyl-6'-one as described in <1> or <2> above,
   wherein an adsorbent is used in the step of obtaining a bipyridine derivative from an acetylpyridine derivative.
   wherein a compound represented by formulae (II) to (V) is added in portions in the step of obtaining a bipyridine derivative from an acetylpyridine derivative.
<4> The process for producing 2,3'-bipyridyl-6'-one as described in <2> above,
   wherein an amide compound represented by formula (XI) is used in the step of hydrolyzing a ketoester derivative represented by formula (IX) or (X): 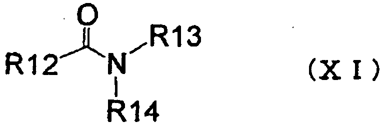 wherein R12 represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a cyano group or a heterocyclic residue;
   each of R13 and R14 independently represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a nitro group, a cyano group, a heterocyclic residue or a halogen atom; and
   two arbitrary groups out of R12 to R14 may combine together to form a ring.
<5> The process for producing 2,3'-bipyridyl-6'-one according, to claim 3, wherein the adsorbent is at least one selected from the group consisting of silica gel, activated carbon, activated clay, RADIOLITE®, activated alumina and acid clay.

### ADVANTAGE OF THE INVENTION

According to the present invention, 2,3'-bipyridyl-6'-one useful in a wide range of fields such as pharmaceutical agents, agricultural chemicals, catalyst ligands, organic electroluminescent devices, electric mobiles, electronic photoreceptors, dyes, liquid crystals and solar cells, particularly useful as an intermediate of pharmaceutical agents for Parkinson's disease, migraine, epilepsy and neurogenic diseases such as multiple sclerosis, can be produced in high purity at low cost on an industrial scale without using an expensive catalyst or special equipment.

### BEST MODE FOR CARRYING OUT THE INVENTION

The present invention is described in detail below.

For illustrating the process of the present invention in detail, one embodiment of the process of the present invention is described below as an example, but contents of the present invention are not limited thereto.

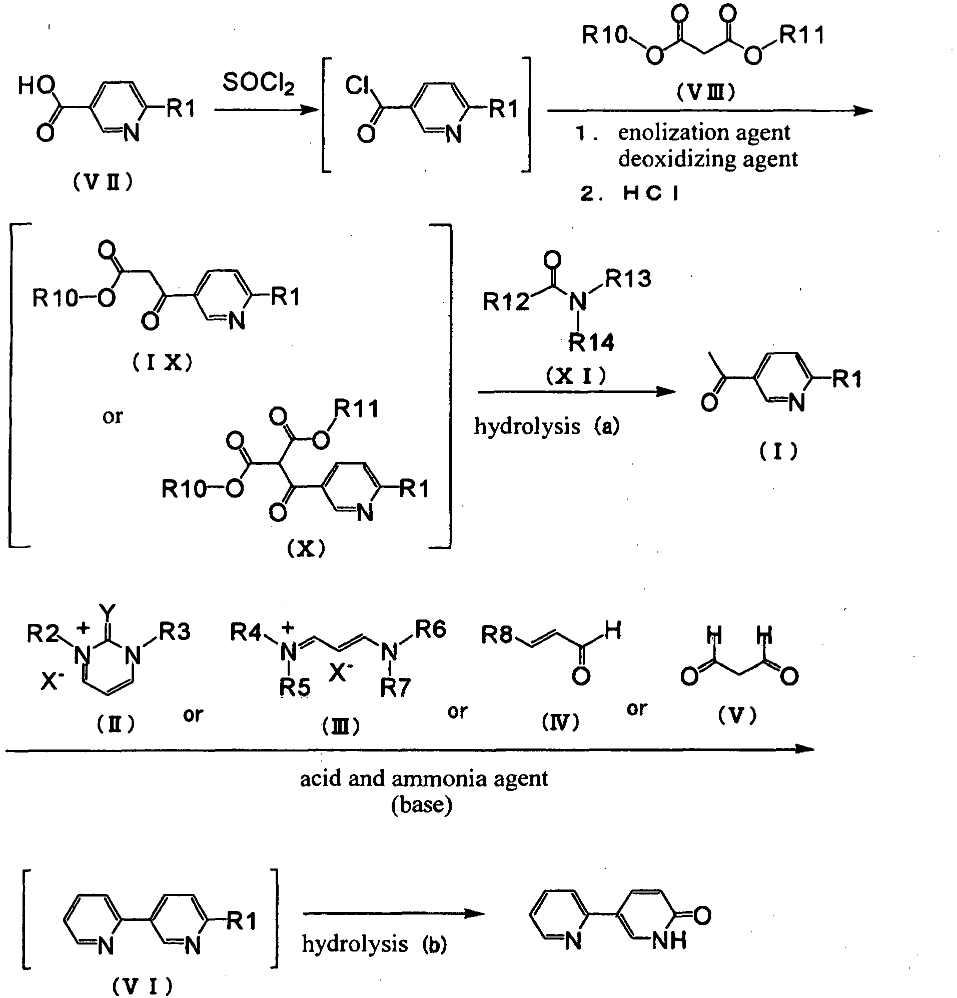

In the formulae above,
R1 represents a hydroxyl group, an alkoxy group or a halogen atom,
each of R2 and R4 independently represents an alkyl group, an alkenyl group, an alkynyl group, an aryl group, a carbonyl group, a sulfonyl group, an amino group, a ureido group, a carbonylamino group, a sulfonylamino group, a cyano group or a heterocyclic residue,
each of R3 and R5 to R7 independently represents an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a nitro group, a cyano group, a heterocyclic residue or a halogen atom,
each of the pair of R4 and R5 and the pair of R6 and R7 may combine together to form a ring,
R8 represents a hydroxyl group, an alkoxy group, an aryloxy group, a carbonyloxy group, a carbonyl group, a sulfonyl group, an amino group, a carbonylamino group or a sulfo group,
X⁻ represents an arbitrary anion,
Y represents an oxygen atom, a sulfur atom, a selenium atom or -N(R9), wherein R9 represents a hydrogen atom, an alkyl group, an alkenyl group, an alkynyl group, an aryl group, a hydroxyl group, a carbonyl group, a sulfonyl group, an amino group or a heterocyclic residue,
each of R10 and R11 independently represents a hydrogen atom, an alkyl group, an alkenyl group, an alkynyl group, an aryl group, a carbonyl group, a heterocyclic residue, an alkali metal atom, an alkaline earth metal atom, a typical metal atom, a transition metal atom or a nonmetallic atom, R10 and R11 may combine to form a ring,
R12 represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a cyano group or a heterocyclic residue,
each of R13 and R14 independently represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a nitro group, a cyano group, a heterocyclic residue or a halogen atom, and
two arbitrary groups out of R12 to R14 may combine together to form a ring.

In the compounds represented by formulae (I) to (XI) of the present invention, the alkyl group represented by R2 to R7 and R9 to R14 indicates a linear, branched or cyclic alkyl group having 1 to 20 carbon atoms, such as methyl, ethyl, propyl, butyl, pentyl, hexyl, octyl, nonyl, decyl, undecyl, dodecyl, tridecyl, tetradecyl, pentadecyl, hexadecyl, heptadecyl, octadecyl, nonadecyl, icosyl, cyclopropyl, cyclobutyl, cyclopentyl, cyclohexyl, cycloheptyl, cyclooctyl, cyclononyl and cyclodecyl.

The alkenyl group represented by R2 to R7 and R9 to R14 indicates a linear, branched or cyclic alkenyl group having 2 to 20 carbon atoms, such as vinyl, allyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, icosenyl, hexadienyl and dodecatrienyl.

The alkynyl group represented by R2 to R7 and R9 to R14 indicates a linear, branched or cyclic alkynyl group having 2 to 20 carbon atoms, such as ethynyl, butynyl, pentynyl, hexynyl, heptynyl, octynyl, nonynyl, cyclooctynyl, cyclononynyl and cyclodecynyl.

The aryl group represented by R2 to R7 and R9 to R14 indicates a 6- to 10-membered monocyclic or polycyclic aryl group such as phenyl, naphthyl, phenanthryl and anthryl.

The alkoxy group represented by R1, R3, R5 to R8 and R12 to R14 indicates an alkoxy group having 1 to 20 carbon atoms, such as methoxy, ethoxy, propoxy, butoxy, pentyloxy, hexyloxy, heptyloxy, octyloxy, nonyloxy, decyloxy, dodecyloxy and octadecyloxy.

The aryloxy group represented by R3, R5 to R8 and R12 to R14 indicates phenoxy, naphthyloxy or the like.

The carbonyloxy group represented by R3, R5 to R8 and R12 to R14 indicates acetyloxy, ethylcarbonyloxy, propylcarbonyloxy, hexylcarbonyloxy, dodecylcarbonyloxy, benzoylcarbonyloxy, naphthylcarbonyloxy or the like.

The carbonyl group represented by R2 to R14 indicates an alkylcarbonyl group such as acetyl, propionyl, butyryl, pentanoyl, hexanoyl, valeryl and octanoyl; an alkoxycarbonyl group such as methoxycarbonyl, ethoxycarbonyl, tert-butoxycarbonyl, n-decyloxycarbonyl and n-hexadecyloxycarbonyl; an aryloxycarbonyl group such as phenoxycarbonyl and naphthyloxycarbonyl; or an alkyl-substituted carbamoyl such as N-methylcarbamoyl, N-(tert-butyl)carbamoyl, N-dodecylcarbamoyl, N-octadecylcarbamoyl, N,N-dimethylcarbamoyl, N,N-dihexylcarbamoyl and N,N-didodecylcarbamoyl.

The sulfonyl group represented by R2 to R9 and R12 to R14 indicates an alkylsulfonyl group such as methylsulfonyl, ethylsulfonyl, propylsulfonyl, butylsulfonyl, pentyl-sulfonyl, hexylsulfonyl, octylsulfonyl, dodecylsulfonyl and hexadecylsulfonyl; an alkoxysulfonyl group such as methoxysulfonyl, ethoxysulfonyl, tert-butoxysulfonyl, n-decyloxysulfonyl and n-hexadecyloxysulfonyl; an aryloxysulfonyl group such as phenoxysulfonyl and naphthyloxysulfonyl; or an alkyl-substituted sulfamoyl group such as N-ethylsulfamoyl, N-(iso-hexyl)sulfamoyl, N-ethylsulfamoyl, N-decylsulfamoyl, N-hexadecylsulfamoyl, N,N-dimethylsulfamoyl, N,N-dibutoxysulfamoyl, N,N-dioctylsulfamoyl and N,N-tetradecylsulfamoyl.

The amino group represented by R2 to R9 and R12 to R14 indicates an amino group; a monosubstituted amino group such as N-methylamino, N-butylamino, N-hexylamino, N-decylamino, N-tetradecylamino, N-octadecylamino, N-phenylamino and N-naphthylamino; or a disubstituted amino group such as N,N-diethylamino, N,N-diheptylamino, N,N-dioctylamino, N,N-diphenylamino and N,N-methylpropylamino.

The carbonylamino group represented by R2 to R8 and R12 to R14 indicates acetylamino, ethylcarbonylamino, tert-butylcarbonylamino, n-octylcarbonylamino, n-hexadecyl-carbonylamino, benzoylamino, naphthoylamino, methoxy-carbonylamino, ethoxycarbonylamino, n-octylcarbonylamino or n-hexadecyloxycarbonylamino.

The sulfonylamino group represented by R2 to R7 and R12 to R14 indicates methylsulfonylamino, ethylsulfonylamino, tert-butylsulfonylamino, n-octadecylsulfonylamino, phenylsulfonylamino, naphthylsulfonylamino, methoxysulfonylamino, iso-propoxysulfonylamino, n-dodecyloxysulfonylamino or n-hexadecyloxysulfonylamino.

The halogen atom represented by R1, R3, R5 to R7, R13 and R14 specifically indicates chlorine atom, bromine atom, iodine atom, fluorine atom or the like.

The heterocyclic residue represented by R2 to R7 and R9 to R14 indicates a 4- to 10-membered monocyclic or bicyclic heterocyclic group containing from 1 to 4 atoms selected from nitrogen, oxygen and sulfur, such as thiophene, furan, pyran, pyridine, pyrrole, pyrazine, azepine, azosine, azonine, azesine, oxazole, thiazole, pyrimidine, pyridazine, triazine, triazole, tetrazole, imidazole, pyrazole, morpholine, thiomorpholine, piperidine, piperazine, quinoline, isoquinoline, indole, isoindole, quinoxaline, phthalazine, quinolizine, quinazoline, quinoxaline, naphthyridine, chromene, benzofuran and benzothiophene.

The alkali metal atom represented by R10 and R11 indicates lithium atom, sodium atom, potassium atom, rubidium atom, cesium atom or the like.

The alkaline earth metal atom represented by R10 and R11 indicates beryllium atom, magnesium atom, calcium atom, strontium atom, barium atom or the like.

The typical metal atom represented by R10 and R11 indicates aluminum atom or the like.

The transition metal atom represented by R10 and R11 indicates scandium atom, titanium atom, manganese atom, iron atom, cobalt atom, copper atom, zinc atom, gallium atom, silver atom, indium atom, tin atom, antimony atom, bismuth atom or the like.

The nonmetallic atom represented by R10 and R11 indicates boron atom, silicon atom, phosphorus atom, sulfur atom, tellurium atom or the like.

X⁻ specifically represents a halogen ion such as fluoride ion, chloride ion, bromide ion and iodide ion; an inorganic acid ion such as sulfate ion, phosphate ion, nitrate ion, tetrafluoroborate ion, hexafluorophosphate ion and perchlorate ion; a Lewis acid-containing ion such as tetrachloroaluminum ion and tetrabromoferrate(III) ion; or an organic acid ion such as acetate ion, lactate ion, citrate ion, benzoate ion, methanesulfonate ion, ethanesulfonate ion, benzenesulfonate ion, toluenesulfonate ion, trifluoroacetate ion, trifluoromethanesulfonate ion, isethionate ion, glucuronate ion, gluconate ion and tetraphenylborate ion.

The ring formed by combining R4 and R5 or combining R6 and R7 indicates an aromatic ring, a saturated ring, a partially saturated ring, a heterocyclic ring or the like, each having 3 to 10 carbon atoms.

The ring formed by combining R10 and R11 indicates a heterocyclic ring having 4 to 10 carbon atoms, such as Meldrum's acid.

The ring formed by combining two arbitrary groups out of R12 to R14 indicates an aromatic ring, a saturated ring, a partially saturated ring, a heterocyclic ring or the like, each having 3 to 10 carbon atoms.

R1 is preferably chlorine atom, bromine atom, an alkoxy group having 1 to 12 carbon atoms, or a hydroxyl group, more preferably chlorine atom, an alkoxy group having 1 to 4 carbon atoms, or a hydroxyl group, still more preferably chlorine atom.

Each of R2 to R7 and R9 is preferably an alkyl group having 1 to 12 carbon atoms, an aryl group having 6 to 20 carbon atoms, or a heterocyclic residue, more preferably an alkyl group having 1 to 6 carbon atoms, or an aryl group having 6 to 12 carbon atoms, still more preferably methyl group.

R8 is preferably an alkoxy group having 1 to 10 carbon atoms, or an amino group.

Each of R10 and R11 is preferably hydrogen atom, an alkyl group having 1 to 12 carbon atoms, an alkali metal atom or an alkaline earth metal atom, more preferably an alkyl group having 1 to 6 carbon atoms, or an alkali metal atom.

R12 is preferably hydrogen atom, an alkyl group having 1 to 12 carbon atoms, an aryl group having 6 to 20 carbon atoms, or a heterocyclic residue, more preferably hydrogen atom or an alkyl group having 1 to 6 carbon atoms.

Each of R13 and R14 is preferably an alkyl group having 1 to 12 carbon atoms, or an aryl group having 6 to 20 carbon atoms, more preferably an alkyl group having 1 to 6 carbon atoms, or an aryl group having 6 to 10 carbon atoms.

X⁻ is preferably chlorine ion, bromine ion, sulfate ion, tetrafluoroborate ion, acetate ion or methanesulfonate ion, more preferably chlorine ion, tetrafluoroborate ion or methanesulfonate ion.

Each of R1 to R14 may further have a substituent, and the substituent is not particularly limited as long as it does not participate in the reaction. Specific examples of the substituent include an alkyl group such as methyl, ethyl, propyl, butyl, pentyl, hexyl, heptyl, octyl, nonyl and decyl; an alkenyl group such as vinyl, allyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, icosenyl, hexadienyl and dodecatrienyl; an alkynyl group such as ethynyl, butynyl, pentynyl, hexynyl, heptynyl, octynyl and nonynyl; a monocyclic or di- to tetracyclic aryl group such as phenyl, naphthyl, phenanthryl and anthryl; an alkoxy group such as methoxy, ethoxy, propoxy, butoxy, pentyloxy, hexyloxy, heptyloxy, octyloxy, nonyloxy and decyloxy; an aryloxy group such as phenoxy and naphthyloxy; a disubstituted amino group such as dimethylamino, N-ethyl-N-phenylamino, diphenylamino and N-phenyl-N-naphthylamino; a nitro group; a heterocyclic residue such as furyl, thienyl and pyridyl; and a halogen atom such as fluorine atom, chlorine atom, bromine atom and iodine atom. Among these, an alkyl group, an aryl group, a heterocyclic residue and a halogen atom are preferred, and an alkyl group and an aryl group are more preferred.

Incidentally, 2,3'-bipyridyl-6'-one of the present invention includes 2,3'-bipyridyl-6'-ol that is a tautomer thereof.

The production process of the present invention is described below.

First, the step of producing nicotinoyl chloride from a nicotinic acid derivative (VII) is described. As to the nicotinic acid derivative for use in the present invention, various types are commercially produced and easily available.

As for the acid-chloridizing agent, various types are commercially produced, but use of thionyl chloride that is inexpensive, easily available and easily handleable is preferred. The amount of thionyl chloride used is from 0.1 to 10 mol, preferably from 0.5 to 5.0 mol, more preferably from 0.8 to 2.0 mol, per mol of the nicotinic acid derivative.

This step may be performed in a solventless system or in the presence of a solvent. In the case of using a solvent, the solvent is not particularly limited in its kind as long as it dose not participate in the reaction, but examples thereof include an ester-based solvent such as methyl acetate, ethyl acetate, isopropyl acetate and butyl acetate; a nitrile-based solvent such as acetonitrile, propionitrile and benzonitrile; and an aromatic solvent such as benzene, toluene, xylene, ethylbenzene, chlorobenzene, dichlorobenzene and mesitylene. The solvent is preferably ethyl acetate, butyl acetate, acetonitrile or toluene, more preferably acetonitrile. Also, two or more kinds of solvents may be mixed and used, and in the case of mixing solvents, the mixing ratio can be arbitrarily selected. The amount of the solvent used is from 0.1 to 100 times by weight, preferably from 1 to 50 times by weight, more preferably from 1.5 to 10 times by weight, based on the nicotinic acid derivative.

The reaction temperature at the acid chloridization is from -20 to 200°C, preferably from 0 to 150°C, more preferably from 50 to 130°C.

The nicotinoyl chloride may be isolated but may also be produced and used by so-called one-pot preparation of using it directly to the subsequent reaction without performing an operation such as purification. The reaction solution after the completion of reaction may be used as it is in the next step, but after excess thionyl chloride is distilled off by concentration, the concentrated solution is preferably used as it is for the next reaction.

Next, the step of condensing the nicotinoyl chloride and a malonic acid derivative (VIII) in the presence of an enolization agent and a deoxidizing agent, and followed by treatment with a hydrochloric acid to obtain a ketoester derivative (IX) or (X) is described.

The reaction proceeds even if an enolization agent is not used, but the enolization agent is preferably added to enhance selectivity of reaction as the case required. Examples of the enolization agent used include a magnesium compound such as magnesium, magnesium chloride, magnesium bromide, magnesium dimethoxide, magnesium diethoxide, magnesium silicate, magnesium oxide, magnesium carbonate, magnesium nitrate, magnesium hydroxide and magnesium sulfate; and a Lewis acid such as zinc chloride, iron chloride, tin chloride, titanium chloride and trifluoroboric acid. Among them, preferably magnesium, magnesium chloride, magnesium bromide, magnesium dimethoxide or magnesium diethoxide, more preferably magnesium chloride is referred. The amount of the enolization agent used is from 0.1 to 20 mol, preferably from 0.5 to 10 mol, more preferably from 1 to 5 mol, per mol of the nicotinic acid derivative.

Examples of the deoxidizing agent used include an organic base such as pyridine, 2-methylpyridine, diethylamine, diisopropylamine, triethylamine, phenylethylamine, isopropylethylamine, methylaniline, tetrabutylammonium hydroxide, 1,8-diazabicyclo[5,4,0]undec-7-ene (hereinafter simply referred to as "DBU") and potassium acetate; an organic metal such as n-butyl lithium and tert-butyl magnesium chloride; an inorganic base such as sodium borohydride, sodium, sodium hydride, calcium oxide, lithium hydroxide, potassium phosphate, sodium carbonate and potassium carbonate; and a metal alkoxide such as potassium tert-butoxide, sodium tert-butoxide and sodium ethoxide. Among them, preferably pyridine, diethylamine, triethylamine or potassium carbonate, more preferably triethylamine is referred. The amount of the deoxidizing agent used is from 0.1 to 20 mol, preferably from 0.5 to 10 mol, more preferably from 1 to 5 mol, per mol of the nicotinic acid derivative.

Specific examples of the malonic acid derivative include dimethyl malonate, diethyl malonate, dipropyl malonate, dibutyl malonate, Meldrum's acid, potassium monoethyl malonate, sodium monoethyl malonate, disodium malonate and malonic acid. Among them, preferably dimethyl malonate, diethyl malonate, Meldrum's acid, potassium monoethyl malonate or sodium monoethyl malonate, more preferably potassium monoethyl malonate or sodium monoethyl malonate is referred. The amount of the malonic acid derivative used is from 0.1 to 20 mol, preferably from 0.8 to 10 mol, more preferably from 1 to 3 mol, per mol of the nicotinic acid derivative.

The temperature at the condensation reaction of the nicotinoyl chloride with a malonic acid derivative is from -20 to 200°C, preferably from 0 to 150°C, more preferably from 20 to 150°C. The temperature in the range from 40 to 90°C is particularly preferred, because reaction terminates within 3 hours immediately after the termination of dropwise addition of the nicotinoyl chloride. Also, before the dropwise addition of nicotinoyl chloride, the enolization agent, deoxidizing agent and malonic acid derivative are preferably mixed with stirring in the presence of a solvent to previously activate the system. The temperature for the activation is from -20 to 200°C, preferably from 0 to 150°C, more preferably from 20 to 120°C, still more preferably from 40 to 90°C. The time required for the activation before the dropwise addition of nicotinoyl chloride is usually from 10 minutes to 24 hours, and the activation terminates within 10 hours in many cases.

The solvent used in this step is not particularly limited as long as it does not participate in the reaction, but examples thereof include an ester-based solvent such as methyl acetate, ethyl acetate, isopropyl acetate and butyl acetate; a nitrile-based solvent such as acetonitrile, propionitrile and benzonitrile; and an aromatic solvent such as benzene, toluene, xylene, ethylbenzene, chlorobenzene, dichlorobenzene and mesitylene. Among them, preferably ethyl acetate, butyl acetate, acetonitrile or toluene, more preferably ethyl acetate is referred. Also, two or more kinds of solvents may be mixed and used, and in the case of mixing solvents, the mixing ratio can be arbitrarily selected. The amount of the solvent used is from 1 to 200 times by weight, preferably from 3 to 100 times by weight, more preferably from 5 to 30 times by weight, based on the nicotinic acid derivative.

After the completion of condensation reaction, decarboxylation is performed to treat with an aqueous hydrochloric acid solution, whereby a ketoester derivative (IX) or (X) is obtained. The concentration of the aqueous hydrochloric acid solution used is usually from 0.1 to 35% v/v, preferably from 1 to 25% v/v, more preferably from 5 to 20% v/v. The amount of the aqueous hydrochloric acid solution used is, in the case where the concentration is 10% v/v, from 0.1 to 200 times by weight, preferably from 1 to 100 times by weight, more preferably from 5 to 50 times by weight, based on the nicotinic acid derivative.

The ketoester derivative (IX) or (X) obtained in this step can be produced and used by so-called one-pot preparation of using it for the subsequent reaction without performing an operation such as isolation or purification. For example, the reaction solution after decarboxylation with an aqueous hydrochloric acid solution is subjected to liquid separation, the organic solvent such as ethyl acetate used for the reaction solvent is neutralized by the washing with an aqueous sodium bicarbonate solution, the resulting solution is concentrated, and the obtained crude product is used in the next reaction step.

The step of hydrolysis reaction (a) of a ketoester derivative (IX) or (X) to produce an acetylpyridine derivative (I) is described below.

The hydrolysis reaction (a) may be performed using a reagent usually employed in hydrolysis, such as acid (e.g., hydrochloric acid, sulfuric acid), base (e.g., aqueous sodium hydroxide solution, sodium ethoxide, ammonia) or dimethylsulfoxide, and is preferably performed in the presence of an amide compound (XI). The amide compound used in the present invention is not particularly limited as long as it does not participate in the reaction. Examples of the amide compound are set forth below.

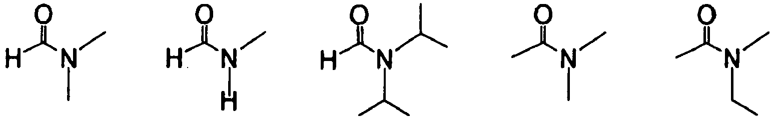

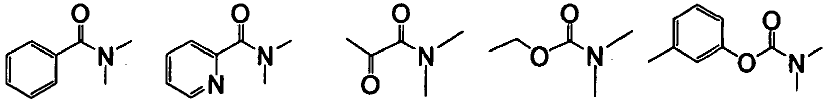

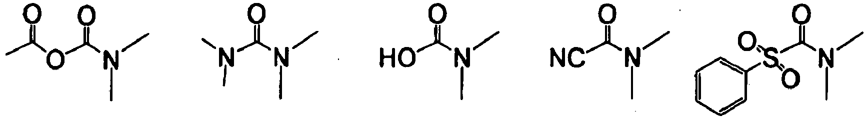

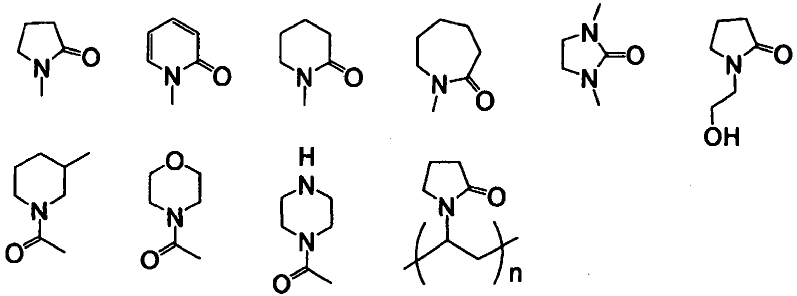

Among them, preferred are N-methylformamide, N,N-dimethylformamide (hereinafter simply referred to as "DMF"), N,N-diisopropylformamide, N-methyl-2-pyrolidinone (hereinafter simply referred to as "NMP"), N,N-dimethylacetamide, N-methyl-2-pyrrolidone, N-acetylpiperazine, N-acetylmorpholine, N-methylpyridone and N-methylpiperidone, more preferred are DMF, NMP, N,N-dimethylacetamide and N-methylformamide.

The amount of the reagent used in the hydrolysis reaction (a) is usually from 0.1 to 100 times by weight, preferably from 0.2 to 80 times by weight, more preferably from 0.5 to 30 times by weight, based on the nicotinic acid derivative.

The hydrolysis reaction (a) is performed in the presence of water. The amount of water used is from 0.001 to 100 times by weight, preferably from 0.01 to 50 times by weight, more preferably from 0.1 to 20 times by weight, based on the nicotinic acid derivative.

The reaction temperature of the hydrolysis reaction (a) is from -20 to 200°C, preferably from 0 to 180°C, more preferably from 50 to 150°C. This reaction usually terminates within 24 hours and in many cases, disappearance of raw materials is confirmed in 1 to 16 hours.

The acetylpyridine derivative (I) obtained in this step can be easily taken out as crystals by adding water after the completion of reaction.

In the present invention, the synthesis for obtaining an acetylpyridine derivative from a nicotinic acid derivative is preferably performed by so-called one-pot preparation of not performing an operation such as isolation or purification in the middle of the course.

The step of producing a bipyridine derivative (VI) by reacting an acetylpyridine derivative (I), a compound represented by formulae (II) to (V) and an ammonia agent in the presence of an acid to form a pyridine ring is described below.

As for compounds represented by formulae (II) to (V), various types are commercially produced and easily available, and the commercial product can be used as it is. Also, the compounds can be easily synthesized by a known method (see, for example, JP-A-2005-213239, JP-A-2003-160550, JP-A-2001-261646 and JP-A-2001-261653).

Specific preferred examples of the compounds represented by formulae (II) to (V) are set forth below.

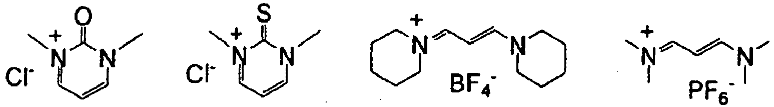

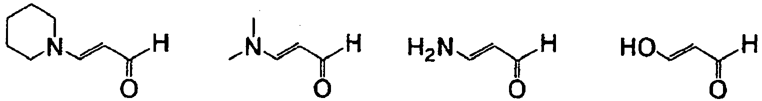

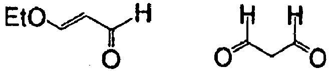

More preferred compounds are 1,3-dimethyl-2-oxo-pyrimidinium chloride and 3-piperidino-2-prop-2-enylidene piperidinium tetrafluoroborate. Above all, 1,3-dimethyl-2-oxo-pyrimidinium chloride is particularly preferred, because this compound can be produced in an industrially advantageous manner by a simple and easy operation.

The amount of the compound represented by formulae (II) to (V) is from 0.5 to 6 mol, preferably from 0.8 to 2.5 mol, more preferably from 1.0 to 2.0 mol, per mol of the acetylpyridine derivative. The compound represented by formulae (II) to (V) is added in portions in the step of obtaining a bipyridine derivative from an acetylpyridine derivative. The amount of the compound for the first addition is from 0.5 to 2.0 mol, preferably from 0.8 to 1.5 mol, more preferably from 1.0 to 1.3 mol, per mol of the acetylpyridine derivative. The remaining amount of the compound may be added in arbitrary parts. The number of additions is from 2 to 10, preferably from 2 to 5, more preferably from 2 to 3.

The timing of second and subsequent additions varies depending on the amount of the compound for the first addition or reaction temperature, but the compound is added when the reaction ratio exceeds 45%, preferably when the reaction ratio exceeds 70%, more preferably when the reaction ratio exceeds 80%.

In the case of using the compound of formulae (III) to (V) in this step, a base is previously added for effecting activation by drawing out a proton of the acetyl group of the acetylpyridine derivative and after the activation, an acid and an ammonium agent are added. The base used is specifically an organic base such as pyridine, 2-methylpyridine, diethylamine, diisopropylamine, triethylamine, phenylethylamine, isopropylethylamine, methylaniline, tetrabutylammonium hydroxide, DBU and potassium acetate; an organic metal such as n-butyl lithium and tert-butyl magnesium chloride; an inorganic base such as sodium borohydride, sodium, potassium hydride and calcium oxide; or a metal alkoxide such as potassium tert-butoxide, sodium tert-butoxide and sodium ethoxide. Among them, preferably potassium tert-butoxide or sodium tert-butoxide, more preferably potassium tert-butoxide is referred. The amount of the base used is from 0.1 to 20 mol, preferably from 0.8 to 10 mol, more preferably from 0.9 to 2 mol, per mol of the acetylpyridine derivative.

The acid used in this step is not particularly limited as long as it does not participate in the reaction. Examples thereof include an inorganic acid such as sulfuric acid, hydrochloric acid and phosphoric acid; an organic acid such as p-toluenesulfonic acid, formic acid, acetic acid, propionic acid and trifluoroacetic acid; and a strongly acidic ion exchange resin such as Amberlite and Amberlist. Among them, preferably formic acid, acetic acid, propionic acid or trifluoroacetic acid, more preferably acetic acid is referred. The amount of the acid used is from 1 to 30 mol, preferably from 2 to 15 mol, more preferably from 3 to 10 mol, still more preferably from 6.1 to 8 mol, per mol of the acetylpyridine derivative.

The ammonia agent used in this step may be used in any form such as ammonia or ammonia salt. Examples thereof include ammonia gas, aqueous ammonia, ammonium chloride, ammonium acetate, ammonium formate, acetamide and sodium amide. The ammonia agent is preferably ammonium chloride, ammonium acetate or ammonium formate, more preferably ammonium acetate. The amount of the ammonia agent used is from 1 to 30 mol, preferably from 2 to 15 mol, more preferably from 3 to 10 mol, per mol of the acetylpyridine derivative. Also, two or more kinds of ammonia agents differing in the form may be mixed and used, and in the case of ammonia agents mixed, the mixing ratio may be arbitrarily selected.

Incidentally, when using the compound of formula (II), the ammonia agent may be added simultaneously with other reagents before the initiation of the reaction or may be added after 3 to 6 hours from the initiation of the reaction but in view of simplicity, the ammonia agent is preferably added simultaneously with other reagents before the initiation of the reaction. In the case of the compounds of formulae (III) to (V), the ammonia agent is added after activating the acetyl group of the acetylpyridine derivative with a base.

In the step of producing a bipyridine derivative, a reaction solvent may not be used or may be used, as the case required. The solvent used is not limited as long as it does not participate in the reaction. Examples thereof include an aromatic solvent such as benzene, toluene, xylene, chlorobenzene and dichlorobenzene; a polar solvent such as pyridine, acetonitrile, N,N-dimethylformamide, N,N-dimethylacetamide and N-methylpyrrolidone; an ether-based solvent such as diethyl ether, diisopropyl ether, dibutyl ether, methyl-tert-butyl ether and tetrahydrofuran (hereinafter simply referred to as "THF"); an ester-based solvent such as methyl acetate, ethyl acetate and butyl acetate; an alcoholic solvent such as methanol, ethanol, n-propyl alcohol, isopropyl alcohol (hereinafter simply referred to as "IPA") and butyl alcohol; and water. Among them, preferably an alcoholic solvent such as methanol, ethanol, n-propyl alcohol, IPA and butyl alcohol, more preferably n-propyl alcohol or IPA is referred. The amount of this reaction solvent used is from 0.1 to 100 times by weight, preferably from 0.5 to 10 times by weight, more preferably from 1 to 3 times by weight, based on the acetylpyridine derivative (I).

The reaction temperature in this step is from -80 to 200°C, preferably from -50 to 150°C, more preferably from -20 to 120°C. The reaction usually terminates within 24 hours.

In the case of producing a bipyridine derivative (VI), a colored component or an inorganic residue is generally produced and therefore, an operation such as isolation and purification is preferably performed using silica gel column, distillation or the like, but use of an adsorbent is more preferred.

In the case of using an adsorbent, the adsorbent is preferably added and stirred after once extracting and concentrating the reaction solution. More specifically, it is preferred that the reaction solution is diluted with an organic solvent such as toluene or ethyl acetate, the organic layer is made basic with sodium hydroxide or the like and extracted, the extract is washed with brine and after concentrating the organic layer to about a half amount, the adsorbent is added and stirred.

Examples of the adsorbent used in the present invention include silica gel, activated carbon, activated clay, diatomaceous earth, activated alumina and acid clay. Among them, preferably silica gel, activated clay or acid clay, more preferably acid clay is referred. The amount of the adsorbent used is usually from 0.01 to 10 times by weight, preferably from 0.1 to 5 times by weight, more preferably from 0.2 to 2 times by weight, based on the theoretical yield of the bipyridine derivative. The treatment time when adding and stirring the adsorbent is usually from 0.1 to 10 hours, preferably from 0.2 to 5 hours, more preferably from 0.5 to 3 hours. The number of treatments is from 1 to 10, preferably from 1 to 5, more preferably from 1 to 3. The treatment temperature is from -20 to 100°C, preferably from 0 to 50°C, more preferably from 10 to 35°C.

In the present invention, it is preferred that after the treatment with adsorbent, the adsorbent is filtered and the objective bipyridine derivative is then extracted into the aqueous layer by using an aqueous hydrochloric acid solution. The bipyridine derivative obtained by this operation can be used directly as an aqueous hydrochloric solution for the next step. The concentration of the aqueous hydrochloric acid solution used is usually from 0.1 to 35% v/v, preferably from 0.5 to 25% v/v, more preferably from 0.1 to 20% v/v. The amount of the aqueous hydrochloric acid solution used is, in the case where the concentration is 10% v/v, from 0.1 to 200 times by weight, preferably from 1 to 100 times by weight, more preferably from 5 to 50 times by weight, based on the acetylpyridine derivative.

The step of hydrolysis reaction (b) of a bipyridine derivative (VI) to produce 2,3'-bipyridyl-6'-one is described below.

The hydrolysis reaction (b) may be performed by using the above-described aqueous hydrochloric acid solution as it is or by additionally addition of an acid. The acid used is not particularly limited as long as it does not participated in the reaction, and examples thereof include a hydrogen acid halide such as hydrochloric acid, hydrobromic acid, hydroiodic acid and hydrofluoric acid; a sulfonic acid such as methanesulfonic acid, benzenesulfonic acid, p-toluenesulfonic acid and trifluoromethanesulfonic acid; a carboxylic acid such as acetic acid and trifluoroacetic acid; sulfuric acid; and nitric acid. Among them, preferably hydrochloric acid, methanesulfonic acid, acetic acid or trifluoroacetic acid, more preferably hydrochloric acid is referred. The amount of the acid used is from 0.01 to 10 times by weight, preferably from 0.1 to 5 times by weight, more preferably from 0.2 to 3 times by weight, based on the acetylpyridine derivative.

The reaction temperature of the hydrolysis reaction (b) is from -20 to 200°C, preferably from 0 to 150°C, more preferably from 50 to 130°C. This reaction usually terminates within 24 hours.

Incidentally, when R1 is a hydroxyl group, that is, in the case of 2,3'-bipyridyl-6'-ol, the hydrolysis is not performed, because this compound is a tautomer of the objective 2,3'-bipyridyl-6'-one.

The obtained 2,3'-bipyridyl-6'-one may be treated by an isolation/purification method usually used for organic compounds. For example, the reaction solution is made neutral with potassium carbonate or the like and extracted with a solvent such as tetrahydrofuran, 1,2-dichloroethane, ethyl acetate or alcohol by a salting-out method, and the extract is concentrated to obtain a crude product. The crude product is further purified, for example, by recrystallization using ethyl acetate, toluene, alcohol, hexane or the like, by column purification using silica gel, or by distillation under reduced pressure. By performing purification using these methods individually or in combination of two or more thereof, the objective compound can be obtained in high purity.

In the present invention, the synthesis for obtaining 2,3'-bipyridyl-6'-one from an acetylpyridine derivative is preferably performed by so-called one-pot preparation of not performing an operation such isolation or purification in the middle of the course.

### EXAMPLES

The present invention is described in greater detail below by referring to Examples, but the present invention is not limited thereto. Incidentally, the evaluation of purity was performed by high-performance liquid chromatography (simply referred to as "HPLC"). HPLC conditions are as follows.

Column: YMC-PACK^{®} ODS AM-302, detection UV: 270 nm, flow rate: 1.0 ml/min, and eluent: methanol/phosphate buffer [pH 6.9] = 40/60.

### Example 1: Synthesis of 3-Acetyl-6-chloropyridine (I-3)

45 g of thionyl chloride (0.378 mol) was added to 150 ml of an acetonitrile solution containing 50 g (0.317 mol) of 6-chloronicotinic acid and the reaction was allowed to proceed at 70°C for 2 hours. After the completion of reaction, the reaction solution was cooled to room temperature, and acetonitrile and excess thionyl chloride were removed under reduced pressure to obtain a crude product (I-1) of 6-chloronicotinoyl chloride.

76 g (0.441 mol) of potassium monoethyl malonate and 91 g (0.951 mol) of magnesium chloride were dissolved in 1,000 ml of ethyl acetate, and 45 g (0.445 mol) of triethylamine was added dropwise thereto, followed by stirring at 70°C for 1 hour. After cooling to 50 to 60°C, the above-obtained crude 6-chloronicotinoyl chloride dissolved in ethyl acetate was added dropwise to the reaction mixture, and the resulting solution was stirred at 50 to 60°C for 1 hour. Subsequent to the completion of reaction, the reaction solution was cooled to 40 to 50°C and a solution obtained by diluting 200 ml of 35% hydrochloric acid with 200 ml of water was added dropwise, followed by stirring for 1 hour. The organic layer obtained by liquid separation was washed with 440 ml of 10% brine and 500 ml of saturated sodium bicarbonate solution and then, ethyl acetate was removed to obtain a crude product (I-2) of ethyl 3-(6-chloro-3-pyridyl)-3-oxopropanate.

Thereafter, the obtained crude ethyl 3-(6-chloro-3-pyridyl)-3-oxopropanate was dissolved in 50 ml of DMF and 10 ml of water and the reaction was allowed to proceed at 110°C for 10 hours. After the completion of reaction, 200 ml of water was added and crystallization was performed at 0 to 5°C for 1 hour to obtain 44.6 g of the objective compound (I-3) (yield: 90.3%). As a result of measurement by HPLC, the purity was 99.6%.

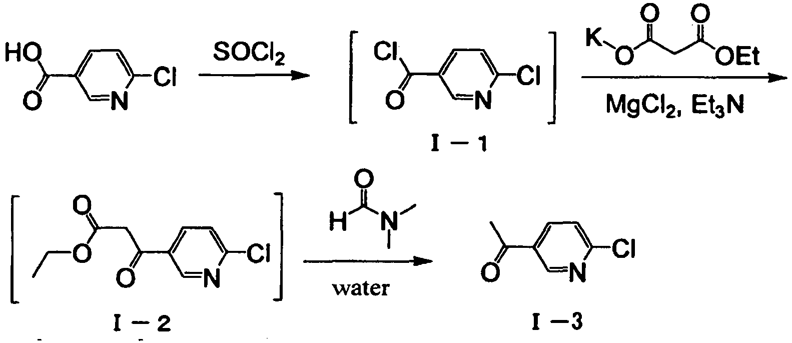

### Examples 2 to 13 and Comparative Examples 1 to 3:

3-Acetyl-6-chloropyridine was synthesized by the same operation as in Example 1 except for changing potassium monoethyl malonate, magnesium chloride and triethylamine used in Example 1 to respective reagents shown in Table 1. The results obtained are shown in Table 1.

**Table 1**

| | Malonic Acid Derivative | Enolization Agent | Deoxidizing agent | Yield (%) | Purity (%) |
|---|---|---|---|---|---|
| Example 1 | potassium monoethyl malonate | MgCl₂ | Et₃N | 90.3 | 99.6 |
| Example 2 | dimethyl malonate | MgCl₂ | Et₃N | 75.2 | 98.8 |
| Example 3 | diethyl malonate | MgCl₂ | Et₃N | 80.3 | 98.3 |
| Example 4 | Meldrum's acid | not added | Et₃N | 70.1 | 97.9 |
| Example 5 | potassium monoethyl malonate | MgO | Et₃N | 68.8 | 99.0 |
| Example 6 | potassium monoethyl malonate | Mg(OEt)₂ | Et₃N | 70.2 | 98.8 |
| Example 7 | potassium monoethyl malonate | MgCl₂ | pyridine | 65.2 | 98.4 |
| Example 8 | potassium monoethyl malonate | not added | DBU | 55.2 | 97.6 |
| Example 9 | potassium monoethyl malonate | not added | tert-BuOK | 40.2 | 97.9 |
| Example 10 | potassium monoethyl malonate | MgCl₂ | K₂CO₃ | 64.4 | 98.3 |
| Example 11 | potassium monoethyl malonate | MgBr₂ | Et₃N | 67.9 | 98.9 |
| Example 12 | sodium monoethyl malonate | Mg(OMe)₂ | Et₃N | 68.3 | 98.8 |
| Example 13 | sodium monoethyl malonate | MgCl₂ | Et₂NH | 64.7 | 98.8 |
| Comparative Example 1 | ethyl acetate | MgCl₂ | Et₃N | not detected | - |
| Comparative Example 2 | ethyl acetate | not added | tert-BuOK | not detected | - |
| Comparative Example 3 | ethyl acetoacetate | MgCl₂ | Et₃N | not detected | - |

The results shown in Table 1 reveal the followings.

According to the process of the present invention, 3-acetyl-6-chloropyridine can be synthesized in high purity and high yield. On the other hand, in Comparative Examples 1 to 3 where acetic acid or ethyl acetoacetate is used in place of a malonic acid derivative, the condensation reaction does not proceed and the objective compound cannot be obtained.

### Examples 14 to 22:

3-Acetyl-6-chloropyridine was synthesized by the same operation as in Example 1 except that the conditions of the hydrolysis performed using DMF at 110°C for 10 hours in Example 1 were changed to the conditions shown in Table 2. The results obtained are shown in Table 2.

**Table 2**

| | Reagent | Reaction Temperature (°C) | Reaction Time (h) | Yield (%) | Purity (%) |
|---|---|---|---|---|---|
| Example 1 | DMF | 110 | 10 | 90.3 | 99.6 |
| Example 14 | DMF | 90 | 16 | 88.7 | 99.4 |
| Example 15 | NMP | 110 | 8 | 80.4 | 98.8 |
| Example 16 | N,N-dimethylacetamide | 110 | 9 | 79.2 | 98.3 |
| Example 17 | N-methylformamide | 90 | 10 | 73.8 | 98.5 |
| Example 18 | HCl | 90 | 10 | 20.0 | 90.3 |
| Example 19 | HCl/THF | 60 | 10 | 60.3 | 92.1 |
| Example 20 | HCl/IPA | 60 | 10 | 68.1 | 91.3 |
| Example 21 | H₂SO₄ | 60 | 10 | 54.2 | 92.3 |
| Example 22 | dimethylsulfoxide | 155 | 10 | 70.9 | 93.2 |

In this way, according to the process using an amide compound of the present invention, 3-acetyl-6-chloropyridine can be synthesized in high yield and high purity. Particularly, it is seen that the processes of Examples 14 to 17 bring about both high yield and high purity and are more preferred, as compared with Examples 18 to 21 where the hydrolysis is performed under acidic conditions. Also, in the processes of Examples 14 to 17, the synthesis can be performed at a lower temperature than in the process using dimethylsulfoxide of Example 22 and special equipment such as high-temperature reactor need not be used, which is advantageous in view of general versatility, in addition to yield and purity.

### Example 23: Synthesis of 2,3'-Bipyridyl-6'-one (I-5)

59 g (0.983 mol) of acetic acid and 74 g (0.960 mol) of ammonium acetate were added to 50 ml of an IPA solution containing 25 g (0.161 mol) of 3-acetyl-6-chloropyridine and 27 g (0.168 mol) of 1,3-dimethyl-2-oxopyrimidinium chloride and the reaction was allowed to proceed at 100°C for 6 hours. Measurement by HPLC revealed that the reaction ratio at this point was 94.3%. The reaction solution was once cooled, 4 g (0.025 mol) of 1,3-dimethyl-2-oxopyrimidinium chloride was added thereto, and the reaction was again allowed to proceed at 100°C for 5 hours.

After the completion of reaction, the reaction solution was cooled to room temperature, 500 ml of toluene was added, and extraction into the toluene layer was effected by making the solution basic with 25% sodium hydroxide solution. The organic layer after liquid separation was washed with 250 ml of water and then with 250 ml of 10% brine and then, the organic layer was concentrated. Subsequently, 125 ml of toluene was added to the concentrated solution and 10 g of acid clay was further added, followed by stirring for 1 hour. The reaction solution was filtered through Celite^{®} to obtain a toluene solution of crude product of 6'-chloro-2,3'-bipyridyl (I-4).

A toluene solution of the obtained crude 6'-chloro-2,3'-bipyridyl was extracted two times with 125 ml of an aqueous 2 mol/l hydrochloric acid solution. Thereafter, 10 ml of an aqueous 35% hydrochloric acid solution was added to the collected aqueous layer and the reaction was allowed to proceed at 100°C for 10 hours. Subsequent to the completion of reaction, the reaction solution was neutralized with potassium carbonate to a pH of 7 to 8 and 150 ml of THF and 5 g of brine were added thereto, followed by stirring. After liquid separation, 150 ml of THF and 10 g of brine were added to the aqueous layer and liquid separation was again performed. The obtained organic layers were combined, concentrated, dissolved by adding 100 ml of ethyl acetate, cooled to 0 to 5°C and crystallized. The crystals were collected by filtration and dried to obtain 17.5 g of the objective compound (yield: 63.2%). As a result of measurement by HPLC, the purity was 99.8%.

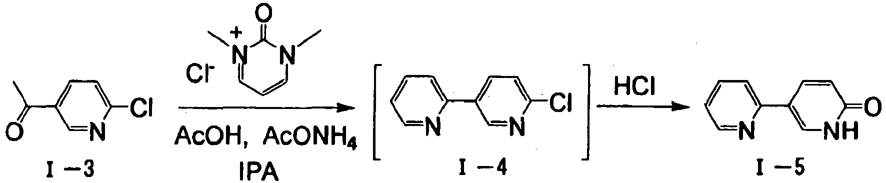

### Examples 24 to 28:

The same operation as in Example 23 was performed except that in Example 23, the number of additions when adding 1,3-dimethyl-2-oxopyrimidinium chloride in portions and the amount added thereof were changed to the conditions shown in Table 3. In Example 27, the addition was only the first addition and after the reaction at 100°C for 6 hours, the subsequent operation was performed in the same manner as in Example 23. In Example 28, the reaction solution after the same reaction as in Example 23 was once cooled, 4 g (0.025 mol) of 1,3-dimethyl-2-oxopyrimidinium chloride was then added, the reaction was allowed to further proceed at 100° for 4 hours, and the subsequent operation was performed in the same manner as in Example 23. Incidentally, the addition in portions was performed after confirming the reaction ratio by HPLC.

The results obtained are shown in Table 3.

**Table 3**

| | Amount Used (mol) <ratio by mol to acetylpyridine derivative> [reaction ratio (%) when added] | | | Yield (%) | Purity (%) |
|---|---|---|---|---|---|
| | first time | second time | third time | | |
| Example 23 | 0.168 | 0.025 | - | 63.2 | 99.8 |
| | <1.05> | <0.15> | | | |
| | [-] | [94.3] | | | |
| Example 24 | 0.193 | 0.048 | - | 60.4 | 99.6 |
| | <1.20> | <0.30> | | | |
| | [-] | [95.5] | | | |
| Example 25 | 0.145 | 0.048 | - | 59.1 | 99.7 |
| | <0.90> | <0.30> | | | |
| | [-] | [87.7] | | | |
| Example 26 | 0.097 | 0.097 | - | 57.6 | 99.0 |
| | <0.60> | <0.60> | | | |
| | [-] | [45.4] | | | |
| Example 27 | 0.193 | - | - | 48.5 | 98.9 |
| | <1.20> | | | | |
| | [-] | | | | |
| Example 28 | 0.168 | 0.025 | 0.025 <0.15> [98.2] | 63.0 | 99.7 |
| | <1.05> | <0.15> | | | |
| | [-] | [93.8] | | | |

As apparent from the results in Table 3, in the process where 1,3-dimethyl-2-oxopyrimidinium chloride is added in portions, the yield and purity are enhanced as compared with the process where the compound is not added in portions.

### Examples 29 to 36:

The same operation as in Example 23 was performed except that in Example 23, the acid clay was changed to the adsorbent shown in Table 4. Incidentally, in Example 36, column purification using silica gel was performed in place of performing the crystallization operation of Example 23. The results obtained are shown in Table 4.

**Table 4**

| | Adsorbent | Purification Treatment | Yield (%) | Purity (%) |
|---|---|---|---|---|
| Example 29 | acid clay | - | 63.2 | 99.8 |
| Example 30 | silica gel | - | 50.5 | 99.8 |
| Example 31 | activated clay | - | 57.1 | 99.4 |
| Example 32 | activated carbon | - | 59.0 | 99.2 |
| Example 33 | activated alumina | - | 49.4 | 99.4 |
| Example 34 | RADIOLITE^{®} | - | 58.5 | 98.7 |
| Example 35 | not added | - | 54.3 | 90.6 |
| Example 36 | not added | column purification by silica gel | 40.3 | 99.8 |

As apparent from the results in Table 4, when the adsorbent is used, the purity is enhanced as compared with the case of not adding the adsorbent or performing the conventional purification method.

### Examples 37 to 41:

2,3'-Bipyridyl-6'-one was synthesized by the same operation as in Example 1 and Example 23 except for using the nicotinic acid derivative shown in Table 4. In Example 37, 2,3'-bipyridyl-6'-one could be directly synthesized and therefore, the operation of hydrolysis in Example 23 was omitted.

**Table 5**

| | Nicotinic Acid Derivative | Yield Until Acetyl Derivative (%) | Yield After Acetyl Derivative (%) | Purity (%) |
|---|---|---|---|---|
| Example 37 | 6-hydroxynicotinic acid | 76.5 | 54.2 | 98.9 |
| Example 38 | 6-bromonicotinic acid | 72.2 | 57.8 | 99.2 |
| Example 39 | 6-methoxynicotinic acid | 85.3 | 54.2 | 98.8 |
| Example 40 | 6-butoxynicotinic acid | 75.7 | 52.6 | 98.8 |
| Example 41 | 6-hexyloxynicotinic acid | 63.7 | 47.2 | 98.4 |

### Example 42: Synthesis of 2,3'-Bipyridyl-6'-one

3 g (0.019 mol) of 3-acetyl-6-chloropyridine and 5.9 g (0.020 mol) of 3-piperidino-2-prop-2-enylidene piperidinium tetrafluoroborate were dissolved in 15 ml of THF and after cooling to 0°C, 2.6 g (0.023 mol) of potassium tert-butoxide was added, followed by stirring at 30°C for 1 hours. Subsequently, 7 ml of acetic acid and 8.9 g (0.115 mol) of ammonium acetate were added and the reaction was allowed to proceed at 100°C for 5 hours. The reaction solution was cooled to 50°C and after further adding 1.0 g (3.4 mmol) of 3-piperidino-2-prop-2-enylidene piperidinium tetrafluoroborate, the reaction was allowed to proceed at 100°C for 4 hours.

Subsequent to the completion of reaction, the reaction solution was cooled to room temperature, 100 ml of toluene was added, and extraction into the toluene layer was effected by making the solution basic with 25% sodium hydroxide solution. The organic layer after liquid separation was washed with 100 ml of water and further with 100 ml of 10% brine and then, the organic layer was concentrated. Thereafter, 10 ml of toluene was added to the concentrated solution and 1 g of acid clay was further added, followed by stirring for 1 hour. The reaction solution was filtered through Celite^{®} to obtain a toluene solution of a crude product (I-4). The hydrolysis was performed by the same method as in Example 23 to obtain 1.9 g of 2,3'-bipyridyl-6'-one (yield: 58.1%). As a result of measurement by HPLC, the purity was 99.1%.

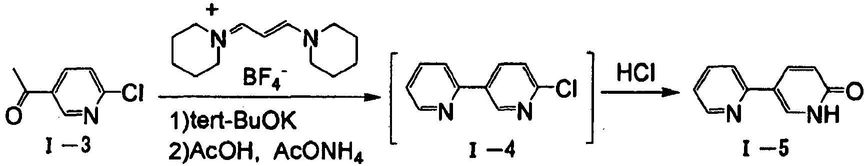

### Examples 43 to 50:

2,3'-Bipyridyl-6'-one was synthesized by the same operation as in Example 42 except that in Example 42, 3-piperidino-2-prop-2-enylidene piperidinium tetrafluoroborate was changed to the compound shown in Table 6. The results obtained are shown in Table 6.

**Table 6**

| | Compound | Yield (%) | Purity (%) |
|---|---|---|---|
| Example 43 | 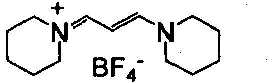 | 58.1 | 99.1 |
| Example 44 | 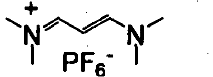 | 58.2 | 98.9 |
| Example 45 | 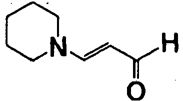 | 56.7 | 99.0 |
| Example 46 | 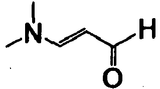 | 53.4 | 99.0 |
| Example 47 | 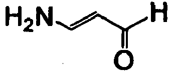 | 57.4 | 98.9 |
| Example 48 | 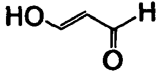 | 50.2 | 98.6 |
| Example 49 | 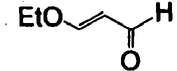 | 55.2 | 98.5 |
| Example 50 | 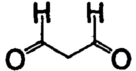 | 56.8 | 99.0 |

### INDUSTRIAL APPLICABILITY

The production process of the present invention enables producing 2,3'-bipyridyl-6'-one in high purity at low cost on an industrial scale without using an expensive catalyst or special equipment and is useful in a wide range of fields such as pharmaceutical agents, agricultural chemicals, catalyst ligands, organic electroluminescent devices, electric mobiles, electronic photoreceptors, dyes, liquid crystals and solar cells, particularly useful for the production of an intermediate of pharmaceutical agents for Parkinson's disease, migraine, epilepsy and neurogenic diseases such as multiple sclerosis.

## Claims

1. A process for producing 2,3'-bipyridyl-6'-one, comprising:
reacting an acetylpyridine derivative represented by formula (I) with at least one of the compounds represented by formulae (II) to (V) to synthesize a bipyridine derivative represented by the following formula (VI); and
hydrolyzing the bipyridine derivative by one-pot preparation: 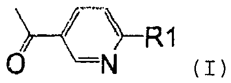 wherein R1 represents a hydroxyl group, an alkoxy group or a halogen atom; 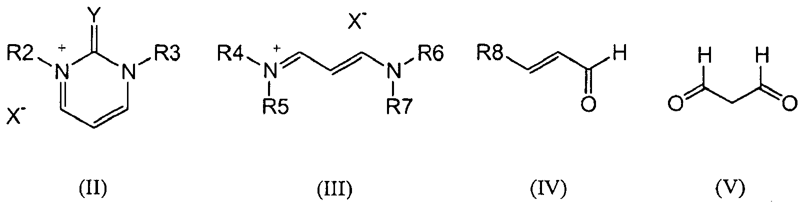 wherein each of R2 and R4 independently represents an alkyl group, an alkenyl group, an alkynyl group, an aryl group, a carbonyl group, a sulfonyl group, an amino group, a ureido group, a carbonylamino group, a sulfonylamino group, a cyano group or a heterocyclic residue;
each of R3 and R5 to R7 independently represents an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, sulfonylamino group, a nitro group, a cyano group, a heterocyclic residue or a halogen atom;
each of the pair of R4 and R5 and the pair of R6 and R7 may combine together to form a ring;
R8 represents a hydroxyl group, an alkoxy group, an aryloxy group, a carbonyloxy group, a carbonyl group, a sulfonyl group, an amino group, a carbonylamino group or a sulfo group;
X⁻ represents an arbitrary anion; and
Y represents an oxygen atom, a sulfur atom, a selenium atom or -N(R9), wherein R9 represents a hydrogen atom, an alkyl group, an alkenyl group, an alkynyl group, an aryl group, a hydroxyl group, a carbonyl group, a sulfonyl group, an amino group or a heterocyclic residue; 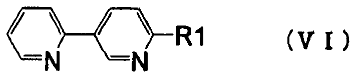 wherein R1 has the same meaning as above, and
wherein at least one of the compounds represented by formulae (II) to (V) is added in portions in the step of obtaining a bipyridine derivative from an acetylpyridine derivative, and
wherein the number of additions of at least one of compounds represented by formulae (II) to (V) is 2 to 10, and
second and subsequent additions of at least one of compounds represented by formulae (II) to (V) are conducted when the reaction ratio exceeds 45%.

2. A process for producing 2,3'-bipyridyl-6'-one, comprising:
a step of converting a nicotinic acid derivative represented by formula (VII) into nicotinoyl chloride;
a step of reacting said nicotinoyl chloride with a malonic acid derivative represented by formula (VIII) to obtain a ketoester derivative represented by formula (IX) or (X);
a step of hydrolyzing said ketoester derivative to obtain an acetylpyridine derivative represented by formula (I);
a step of reacting said acetylpyridine derivative with at least one of compounds represented by formulae (II) to (V) to obtain a bipyridine derivative represented by formula (VI); and
a step of hydrolyzing said bipyridine derivative,
wherein each of the series of the steps from said nicotinic acid derivative to said acetylpyridine derivative and the series of the steps from said acetylpyridine derivative to 2,3'-bipyridyl-6'-one is performed by one-pot preparation: 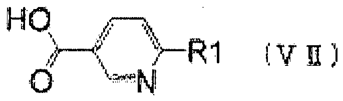 wherein R1 represents a hydroxyl group, an alkoxy group or a halogen atom; 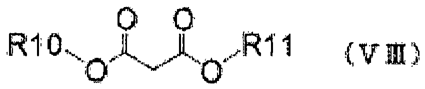
wherein each of R10 and R11 independently represents a hydrogen atom, an alkyl group, an alkenyl group, an alkynyl group, an aryl group, a carbonyl group, a heterocyclic residue, an alkali metal atom, an alkaline earth metal atom, a typical metal atom, a transition metal atom or a nonmetallic atom, and R10 and R11 may combine to form a ring; 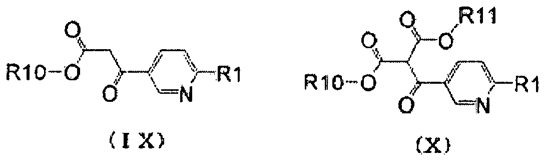 wherein R1, R10 and R11 have the same meanings as above, and
wherein at least one of the compounds represented by formulae (II) to (V) is added in portions in the step of obtaining a bipyridine derivative from an acetylpyridine derivative, and
wherein the number of additions of at least one of compounds represented by formulae (II) to (V) is 2 to 10, and
second and subsequent additions of at least one of compounds represented by formulae (II) to (V) are conducted when the reaction ratio exceeds 45%.

3. The process for producing 2,3'-bipyridyl-6'-one according to claim 1 or 2,
wherein an adsorbent is used in the step of obtaining a bipyridine derivative from an acetylpyridine derivative.

4. The process for producing 2,3'-bipyridyl-6'-one according to claim 2,
wherein an amide compound represented by formula (XI) is used in the step of hydrolyzing a ketoester derivative represented by formula (IX) or (X): 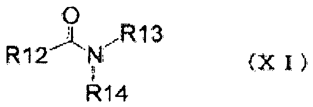 wherein R12 represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a cyano group or a heterocyclic residue;
each of R13 and R14 independently represents a hydrogen atom, an alkyl group, an alkenyl group, an alkynyl group, an aryl group, a hydroxyl group, an alkoxy group, an aryloxy group, a carbonyloxy group, a carbonyl group, a sulfonyl group, an amino group, a ureido group, a carbonylamino group, a sulfonylamino group, a nitro group, a cyano group, a heterocyclic residue or a halogen atom; and
two arbitrary groups out of R12 to R14 may combine together to form a ring.

5. The process for producing 2, 3'-bipyridyl-6'-one according, to claim 3,
wherein the adsorbent is at least one selected from the group consisting of silica gel, activated carbon, activated clay, diatomaceous earth, activated alumina and acid clay.

## Patentansprüche

1. Verfahren zur Herstellung von 2,3'-Bipyridyl-6'-on, umfassend:
Umsetzen eines Acetylpyridinderivats der Formel (I) mit mindestens einer der Verbindungen der Formeln (II) bis (V), um ein Bipyridinderivat der nachstehenden Formel(VI) zu synthetisieren; und
Hydrolysieren des Bipyridinderivats durch Ein-Topf-Herstellung: 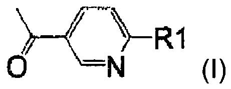 worin R1 eine Hydroxylgruppe, eine Alkoxygruppe oder ein Halogenatom darstellt; 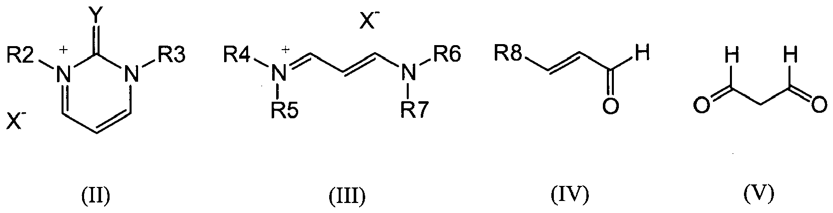 worin R2 und R4 jeweils unabhängig eine Alkylgruppe, eine Alkenylgruppe, eine Alkinylgruppe, eine Arylgruppe, eine Carbonylgruppe, eine Sulfonylgruppe, eine Aminogruppe, eine Ureidogruppe, eine Carbonylaminogruppe, eine Sulfonylaminogruppe, eine Cyanogruppe oder einen heterocyclischen Rest darstellen;
R3 und R5 bis R7 jeweils unabhängig eine Alkylgruppe, eine Alkenylgruppe, eine Alkinylgruppe, eine Arylgruppe, eine Hydroxylgruppe, eine Alkoxygruppe, eine Aryloxygruppe, eine Carbonyloxygruppe, eine Carbonylgruppe, eine Sulfonylgruppe, eine Aminogruppe, eine Ureidogruppe, eine Carbonylaminogruppe, eine Sulfonylaminogruppe, eine Nitrogruppe, eine Cyanogruppe, einen heterocyclischen Rest oder ein Halogenatom darstellen;
jedes der Paare von R4 und R5 und der Paare von R6 und R7 miteinander verbunden sein kann, um einen Ring zu bilden;
R8 eine Hydroxylgruppe, eine Alkoxygruppe, eine Aryloxygruppe, eine Carbonyloxygruppe, eine Carbonylgruppe, eine Sulfonylgruppe, eine Aminogruppe, eine Carbonylaminogruppe oder eine Sulfogruppe darstellt;
X- ein beliebiges Anion darstellt; und
Y ein Sauerstoffatom, ein Schwefelatom, ein Selenatom oder -N(R9) darstellt, worin R9 ein Wasserstoffatom, eine Alkylgruppe, eine Alkenylgruppe, eine Alkinylgruppe, eine Arylgruppe, eine Hydroxylgruppe, eine Carbonylgruppe, eine Sulfonylgruppe, eine Aminogruppe oder einen heterocyclischen Rest darstellt; 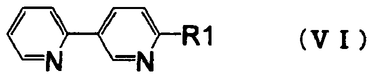 worin R1 die gleiche Bedeutung hat wie oben, und
wobei mindestens eine der Verbindungen der Formeln (II) bis (V) in dem Schritt, in dem ein Bipyridinderivat aus einem Acetylpyridinderivat erhalten wird, portionsweise zugefügt wird und
wobei die Anzahl an Zugaben von mindestens einer der Verbindungen der Formeln (II) bis (V) 2 bis 10 beträgt, und
eine zweite und nachfolgende Zugaben von mindestens einer der Verbindungen der Formeln (II) bis (V) durchgeführt werden, wenn das Reaktionsverhältnis 45 % übersteigt.

2. Verfahren zur Herstellung von 2,3'-Bipyridyl-6'-on, umfassend:
einen Schritt, in dem ein Nikotinsäurederivat der Formel (VII) zu Nikotinoylchlorid umgewandelt wird;
einen Schritt, in dem das Nikotinoylchlorid mit einem Malonsäurederivat der Formel (VIII) umgesetzt wird, um ein Ketoesterderivat der Formel (IX) oder (X) zu erhalten;
einen Schritt, in dem das Ketoesterderivat hydrolysiert wird, um ein Acetylpyridinderivat der Formel (I) zu erhalten;
einen Schritt, in dem das Acetylpyridinderivat mit mindestens einer der Verbindungen der Formeln (II) bis (V) umgesetzt wird, um ein Bipyridinderivat der Formel (VI) zu erhalten; und
einen Schritt, in dem das Bipyridinderivat hydrolysiert wird,
wobei die Reihe der Schritte vom Nikotinsäurederivat zum Acetylpyridinderivat und die Reihe der Schritte vom Acetylpyridinderivat zum 2,3'-Bipyridyl-6'-on jeweils durch eine Ein-Topf-Herstellung durchgeführt wird: 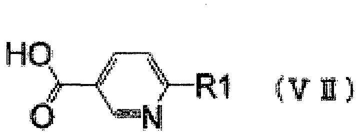 worin R1 eine Hydroxylgruppe, eine Alkoxygruppe oder ein Halogenatom darstellt; 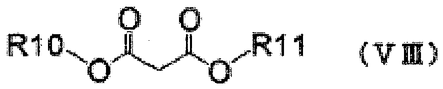
worin R10 und R11 jeweils unabhängig ein Wasserstoffatom, eine Alkylgruppe, eine Alkenylgruppe, eine Alkinylgruppe, eine Arylgruppe, eine Carbonylgruppe, einen heterocyclischen Rest, ein Alkalimetallatom, ein Erdalkalimetallatom, ein typisches Metallatom, ein Übergangsmetallatom oder ein nicht-metallisches Atom darstellen, und R10 und R11 verbunden sein können, um einen Ring zu bilden; 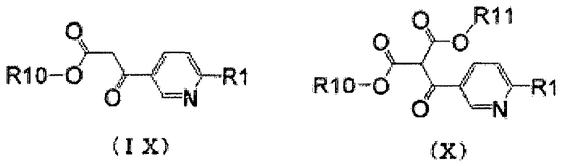 worin R1, R10 und R11 die gleichen Bedeutungen haben wie oben, und
wobei mindestens eine der Verbindungen der Formeln (II) bis (V) in dem Schritt, in dem ein Bipyridinderivat aus einem Acetylpyridinderivat erhalten wird, portionsweise zugefügt wird und
wobei die Anzahl an Zugaben von mindestens einer der Verbindungen der Formeln (II) bis (V) 2 bis 10 beträgt, und
eine zweite und nachfolgende Zugaben von mindestens einer der Verbindungen der Formeln (II) bis (V) durchgeführt werden, wenn das Reaktionsverhältnis 45 % übersteigt.

3. Verfahren zur Herstellung von 2,3'-Bipyridyl-6'-on gemäss Anspruch 1 oder 2, wobei in dem Schritt, in dem ein Bipyridinderivat aus einem Acetylpyridinderivat erhalten wird, ein Adsorptionsmittel verwendet wird.

4. Verfahren zur Herstellung von 2,3'-Bipyridyl-6'-on gemäss Anspruch 2,
wobei in dem Schritt, in dem ein Ketoesterderivat der Formel (IX) oder (X) hydrolysiert wird, eine Amidverbindung der Formel (XI) verwendet wird: 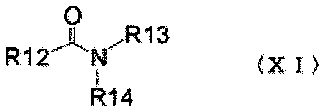 worin R12 ein Wasserstoffatom, eine Alkylgruppe, eine Alkenylgruppe, eine Alkinylgruppe, eine Arylgruppe, eine Hydroxylgruppe, eine Alkoxygruppe, eine Aryloxygruppe, eine Carbonyloxygruppe, eine Carbonylgruppe, eine Sulfonylgruppe, eine Aminogruppe, eine Ureidogruppe, eine Carbonylaminogruppe, eine Sulfonylaminogruppe, eine Cyanogruppe oder einen heterocyclischen Rest darstellt;
R13 und R14 jeweils unabhängig ein Wasserstoffatom, eine Alkylgruppe, eine Alkenylgruppe, eine Alkinylgruppe, eine Arylgruppe, eine Hydroxylgruppe, eine Alkoxygruppe, eine Aryloxygruppe, eine Carbonyloxygruppe, eine Carbonylgruppe, eine Sulfonylgruppe, eine Aminogruppe, eine Ureidogruppe, eine Carbonylaminogruppe, eine Sulfonylaminogruppe, eine Nitrogruppe, eine Cyanogruppe, einen heterocyclischen Rest oder ein Halogenatom darstellen; und
zwei beliebige Gruppen aus R12 bis R14 miteinander verbunden sein können, um einen Ring zu bilden.

5. Verfahren zur Herstellung von 2,3'-Bipyridyl-6'-on gemäss Anspruch 3, wobei das Adsorptionsmittel mindestens eines, ausgewählt aus der Gruppe bestehend aus Silicagel, Aktivkohle, aktiviertem Ton, Kieselgur, aktiviertem Aluminiumoxid und saurem Ton, ist.

## Revendications

1. Procédé pour produire de la 2,3'-bipyridyl-6'-one, comprenant :
la réaction d'un dérivé d'acétylpyridine représenté par la formule (I) avec au moins l'un des composés représentés par les formules (II) à (V) pour que soit synthétisé un dérivé de bipyridine représenté par la formule (VI) suivante ; et
l'hydrolyse du dérivé de bipyridine par préparation monotope : 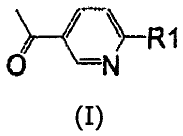 où R1 représente un groupe hydroxyle, un groupe alcoxy ou un atome d'halogène ; 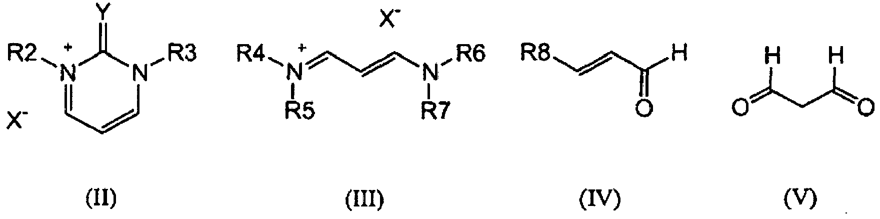 où chacun de R2 et R4 représente indépendamment un groupe alkyle, un groupe alcényle, un groupe alcynyle, un groupe aryle, un groupe carbonyle, un groupe sulfonyle, un groupe amino, un groupe uréido, un groupe carbonylamino, un groupe sulfonylamino, un groupe cyano ou un résidu hétérocyclique ;
chacun de R3 et R5 à R7 représente indépendamment un groupe alkyle, un groupe alcényle, un groupe alcynyle, un groupe aryle, un groupe hydroxyle, un groupe alcoxy, un groupe aryloxy, un groupe carbonyloxy, un groupe carbonyle, un groupe sulfonyle, un groupe amino, un groupe uréido, un groupe carbonylamino, un groupe sulfonylamino, un groupe nitro, un groupe cyano, un résidu hétérocyclique ou un atome d'halogène ;
chacune parmi la paire R4 et R5 et la paire R6 et R7 peut se combiner pour former un cycle ;
R8 représente un groupe hydroxyle, un groupe alcoxy, un groupe aryloxy, un groupe carbonyloxy, un groupe carbonyle, un groupe sulfonyle, un groupe amino, un groupe carbonylamino ou un groupe sulfo ;
X⁻ représente un anion arbitraire ; et
Y représente un atome d'oxygène, un atome de soufre, un atome de sélénium ou - N(R9) où R9 représente un atome d'hydrogène, un groupe alkyle, un groupe alcényle, un groupe alcynyle, un groupe aryle, un groupe hydroxyle, un groupe carbonyle, un groupe sulfonyle, un groupe amino ou un résidu hétérocyclique ; 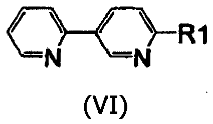 où R1 a la même signification que ci-dessus, et
dans lequel au moins l'un des composés représentés par les formules (II) à (V) est ajouté par portions dans l'étape d'obtention d'un dérivé de bipyridine à partir d'un dérivé d'acétylpyridine, et
dans lequel le nombre d'additions d'au moins l'un des composés représentés par les formules (II) à (V) est de 2 à 10, et
la deuxième addition et les additions suivantes d'au moins l'un des composés représentés par les formules (II) à (V) sont effectuées quand le taux de réaction dépasse 45 %.

2. Procédé pour produire de la 2,3'-bipyridyl-6'-one, comprenant :
une étape de conversion d'un dérivé d'acide nicotinique représenté par la formule (VII) en chlorure de nicotinoyle ;
une étape de réaction dudit chlorure de nicotinoyle avec un dérivé d'acide malonique représenté par la formule (VIII) pour que soit obtenu un dérivé de cétoester représenté par la formule (IX) ou (X) ;
une étape d'hydrolyse dudit dérivé de cétoester pour que soit obtenu un dérivé d'acétylpyridine représenté par la formule (I) ;
une étape de réaction dudit dérivé d'acétylpyridine avec au moins l'un des composés représentés par les formules (II) à (V) pour que soit obtenu un dérivé de bipyridine représenté par la formule (VI) ; et
une étape d'hydrolyse dudit dérivé de bipyridine,
dans lequel chacune de la série d'étapes allant dudit dérivé d'acide nicotinique audit dérivé d'acétylpyridine et de la série d'étapes allant dudit dérivé d'acétylpyridine à la 2,3'-bipyridyl-6'-one est effectuée par préparation monotope : 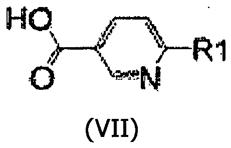 où R1 représente un groupe hydroxyle, un groupe alcoxy ou un atome d'halogène ; 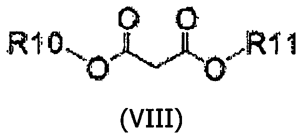 où chacun de R10 et R11 représente indépendamment un atome d'hydrogène, un groupe alkyle, un groupe alcényle, un groupe alcynyle, un groupe aryle, un groupe carbonyle, un résidu hétérocyclique, un atome de métal alcalin, un atome de métal alcalino-terreux, un atome de métal typique, un atome de métal de transition ou un atome non métallique, et R10 et R11 peuvent se combiner pour former un cycle ; 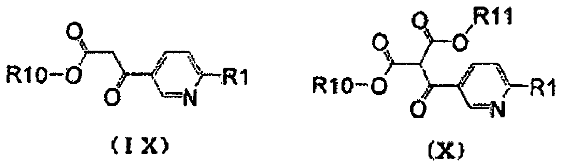 où R1, R10 et R11 ont les mêmes significations que ci-dessus, et
dans lequel au moins l'un des composés représentés par les formules (II) à (V) est ajouté par portions dans l'étape d'obtention d'un dérivé de bipyridine à partir d'un dérivé d'acétylpyridine, et
dans lequel le nombre d'additions d'au moins l'un des composés représentés par les formules (II) à (V) est de 2 à 10, et
la deuxième addition et les additions suivantes d'au moins l'un des composés représentés par les formules (II) à (V) sont effectuées quand le taux de réaction dépasse 45 %.

3. Procédé pour produire de la 2,3'-bipyridyl-6'-one selon la revendication 1 ou 2, dans lequel un adsorbant est utilisé dans l'étape d'obtention d'un dérivé de bipyridine à partir d'un dérivé d'acétylpyridine.

4. Procédé pour produire de la 2,3'-bipyridyl-6'-one selon la revendication 2, dans lequel un composé amide représenté par la formule (XI) est utilisé dans l'étape d'hydrolyse d'un dérivé de cétoester représenté par la formule (IX) ou (X) : 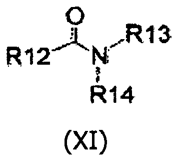 dans laquelle R12 représente un atome d'hydrogène, un groupe alkyle, un groupe alcényle, un groupe alcynyle, un groupe aryle, un groupe hydroxyle, un groupe alcoxy, un groupe aryloxy, un groupe carbonyloxy, un groupe carbonyle, un groupe sulfonyle, un groupe amino, un groupe uréido, un groupe carbonylamino, un groupe sulfonylamino, un groupe cyano ou un résidu hétérocyclique ;
chacun de R13 et R14 représente indépendamment un atome d'hydrogène, un groupe alkyle, un groupe alcényle, un groupe alcynyle, un groupe aryle, un groupe hydroxyle, un groupe alcoxy, un groupe aryloxy, un groupe carbonyloxy, un groupe carbonyle, un groupe sulfonyle, un groupe amino, un groupe uréido, un groupe carbonylamino, un groupe sulfonylamino, un groupe nitro, un groupe cyano, un résidu hétérocyclique ou un atome d'halogène ; et
deux groupes arbitraires parmi R12 à R14 peuvent se combiner ensemble pour former un cycle.

5. Procédé pour produire de la 2,3'-bipyridyl-6'-one selon la revendication 3, dans lequel l'adsorbant est au moins un adsorbant choisi dans le groupe constitué par le gel de silice, le charbon activé, l'argile activée, la terre de diatomées, l'alumine activée et l'argile acide.
